# FASCICULE DE BREVET EUROPEEN

(11) **EP 3 572 030 B1**
(45) Date de publication et mention de la délivrance du brevet: **02.11.2022**
(21) Numéro de dépôt: 18305628.2
(22) Date de dépôt: 22.05.2018
(51) Int. Cl.: A61C 7/00, A61B 5/00, A61C 9/00, A61C 1/08, G06T 7/00, A61B 34/10, A61B 34/20

(54) **PROCEDE D'ANALYSE D'UNE SITUATION DENTAIRE**
VERFAHREN ZUR ANALYSE EINER ZAHNSITUATION
METHOD FOR ANALYSING A DENTAL SITUATION

(43) Date de publication de la demande: 27.11.2019
(73) Titulaire: Dental Monitoring, 75008 Paris (FR)
(72) Inventeur: SALAH, Philippe, 75020 PARIS (FR); PELLISSARD, Thomas, 92110 CLICHY (FR); GHYSELINCK, Guillaume, 59169 CANTIN (FR); DEBRAUX, Laurent, 75020 PARIS (FR)
(74) Mandataire: Nony

(56) Documents cités:
- EP-A1- 3 050 534
- KR-A- 20180 008 532
- US-A1- 2014 147 807
- US-A1- 2016 135 925
- US-A1- 2016 220 105
- US-A1- 2017 065 379
- US-A1- 2017 281 313
- US-A1- 2017 319 293
- US-A1- 2018 125 610

## Description

### Domaine technique

La présente invention concerne l'analyse d'une situation dentaire d'un patient, à des fins de simulation.

### Etat de la technique

L'efficacité d'un traitement dentaire est directement liée au respect de la prescription médicale par le patient. A défaut d'une bonne observance, la situation dentaire peut empirer, ce qui entraîne un risque supplémentaire pour le patient, mais également des coûts pour la Sécurité Sociale ou les organismes de financement des soins.

La mauvaise observance d'un traitement rend également plus difficiles les études cliniques, en particulier si le patient ne déclare pas les périodes de non observance.

Il existe donc un besoin pour un procédé permettant d'améliorer l'observance.

Un but de l'invention est de répondre, au moins partiellement, à ces besoins.

On connaît de US2017/0319293 un procédé permettant à un dentiste de simuler le résultat d'un traitement orthodontique sur un écran, par exemple d'une tablette du dentiste, afin que le patient puisse le visualiser.

### Résumé de l'invention

L'invention propose un procédé d'analyse d'une situation dentaire réelle d'un patient, à des fins de simulation, le procédé comportant les étapes successives suivantes :
A) à un instant actualisé, acquisition d'une image actualisée représentant une scène dentaire réelle telle qu'observée par un opérateur ;
B) détermination d'une scène dentaire virtuelle en fonction de la représentation de la scène dentaire réelle sur l'image actualisée, la scène dentaire virtuelle comportant une représentation de l'arcade du patient telle qu'anticipée pour un instant de simulation futur et/ou une représentation d'un appareil orthodontique porté par les dents de l'arcade telle qu'anticipée pour un instant de simulation futur, l'instant de simulation étant postérieur à l'instant actualisé de plus de 1 jour ;
C) affichage de l'image actualisée sur un écran et présentation de la scène dentaire virtuelle en superposition avec la représentation de la scène dentaire réelle sur l'image actualisée affichée sur l'écran, en transparence ou non sur ladite représentation,
l'acquisition étant effectuée au moyen d'un téléphone portable ou d'une tablette, procédé dans lequel le patient acquiert ladite image actualisée à l'étape A), et observe l'écran à l'étape C).

Dans un mode de réalisation particulier, l'étape B) consiste en une simulation, pour un instant de simulation, de la scène dentaire réelle représentée sur l'image actualisée, puis en une détermination d'une scène dentaire virtuelle en fonction de ladite simulation.

Comme on le verra plus en détail dans la suite de la description, un procédé selon l'invention permet donc à l'opérateur d'observer simultanément la scène dentaire virtuelle en superposition avec la représentation de la scène dentaire réelle sur l'image actualisée.

Cette superposition facilite considérablement l'analyse, par l'opérateur, des différences entre la scène dentaire réelle et la scène dentaire virtuelle. Elle permet également d'apporter des informations relatives à la scène dentaire réelle et de les disposer en fonction de la représentation de la scène dentaire réelle sur l'image actualisée. La qualité de la transmission de ces informations à l'opérateur en est améliorée. Elle permet de simuler de manière réaliste une situation dentaire, par exemple de simuler un traitement esthétique.

Le procédé étant mis en œuvre par le patient ; il permet de simuler de manière réaliste l'effet d'un traitement dentaire ou d'une modification d'un traitement dentaire, ce qui améliore l'observance.

Un procédé selon l'invention peut encore comporter une ou plusieurs des caractéristiques optionnelles suivantes :
- à l'étape B), la scène dentaire virtuelle comporte
   - la représentation d'un ou plusieurs éléments physiques qui ne sont pas dans la scène dentaire réelle, et/ou
   - un indicateur qui ne représente pas un élément physique de la scène dentaire réelle, de préférence plusieurs tels indicateurs ;
- à l'étape B), on identifie un élément de la scène dentaire virtuelle qui ne respecte pas un critère d'évaluation, et on met en évidence cet élément dans la scène dentaire virtuelle et/ou on ajoute à la scène dentaire virtuelle un message relatif audit élément ;
- le critère d'évaluation est relatif à l'état de santé du patient et/ou à un coût pour un traitement dentaire ;
- le message est une consigne à suivre par le patient ;
- à l'étape B), on détermine la nature et/ou l'emplacement et/ou l'apparence d'un élément de la scène dentaire virtuelle, et notamment d'un indicateur, en fonction du contexte, et en particulier en fonction de la distance à un autre élément de la scène dentaire virtuelle et/ou en fonction d'un objectif poursuivi ;
- à l'étape B), on détermine la scène dentaire virtuelle de manière qu'elle comporte des informations, notamment textuelles ou graphiques, sur un emplacement cible, notamment pour un appareil orthodontique ou un article décoratif ;
- à l'étape C), on présente la scène dentaire virtuelle sur l'image actualisée de manière que les représentations d'éléments physiques réels de la scène dentaire virtuelle se superposent, de manière réaliste, avec les représentations desdits éléments physiques réels sur l'image actualisée, respectivement.

De préférence, l'écran et la caméra sont immobilisés par rapport à l'opérateur.

A l'étape C), la scène dentaire virtuelle est présentée, en superposition avec la représentation de la scène dentaire réelle sur l'image actualisée, sur un écran opaque, c'est-à-dire à travers lequel l'opérateur ne peut pas voir.

L'écran est celui d'un téléphone portable ou d'une tablette.

De préférence, l'opérateur manipule un téléphone portable pour acquérir ladite image actualisée et visualiser la scène dentaire réelle et la scène dentaire virtuelle.

Dans un mode de réalisation, à l'étape B), la scène dentaire virtuelle est déterminée en fonction d'une valeur d'au moins un paramètre de traitement modifiable par interaction avec ledit téléphone portable. De préférence, le paramètre de traitement est relatif à un port d'un appareil orthodontique par le patient et/ou au respect d'une consigne de traitement par le patient.

Un procédé selon l'invention peut faciliter les décisions du patient, en particulier parce qu'il lui permet, en choisissant l'instant de simulation, de visualiser l'effet, sur son apparence, des différentes options de traitement possibles. Le procédé peut être également un outil pédagogique efficace pour améliorer l'observance.

### Applications

Un procédé selon l'invention peut être en particulier utilisé à des fins non thérapeutiques, en particulier à des fins de recherche, par exemple pour évaluer l'efficacité d'un traitement ou d'un appareil orthodontique, ou à des fins esthétiques, ou à des fins pédagogiques.

La scène dentaire virtuelle est relative à la scène dentaire réelle telle que simulée à un instant de simulation. En particulier, la scène dentaire virtuelle peut simuler la scène dentaire réelle à l'instant de simulation. La simulation est de préférence obtenue au moyen d'un ordinateur, de préférence au moyen d'un algorithme d'intelligence artificielle.

Le procédé selon l'invention est alors particulièrement utile pour qu'un patient puisse visualiser une situation dentaire à un instant de simulation postérieur à l'instant actualisé, ou visualiser l'évolution d'une telle situation dentaire en faisant varier l'instant de simulation.

Dans un mode de réalisation, on détermine la scène dentaire virtuelle au moyen d'un outil de simulation dynamique configuré pour fournir au moins un élément de la scène dentaire virtuelle en fonction d'un instant de simulation déterminé, en particulier en fonction d'un instant de simulation postérieur à l'instant actualisé.

Le procédé selon l'invention peut comporter une ou plusieurs des caractéristiques optionnelles suivantes :
- l'instant de simulation est par exemple postérieur de plus de 10 jours ou 100 jours, à l'instant actualisé ;
- l'instant de simulation est déterminé avant l'étape A) ou avant l'étape B), de préférence par interaction de l'opérateur avec un écran ;
- ledit écran
   - est un écran tactile et on modifie l'intervalle temporel entre l'instant actualisé et l'instant de simulation par interaction, de préférence par glissement, d'un doigt sur ledit écran, et/ou
   - comporte un champ de saisie de l'instant de simulation ;
- on règle l'instant de simulation en modifiant la position d'un curseur représenté sur l'écran ;
- à l'étape B), on détermine la nature et/ou l'emplacement et/ou l'apparence d'un élément de la scène dentaire virtuelle, et notamment d'un indicateur, en fonction du contexte, et en particulier en fonction de la distance à un autre élément de la scène dentaire virtuelle et/ou en fonction d'un objectif poursuivi, et/ou en fonction de l'instant de simulation ;
- à l'étape B), on détermine, pour l'instant de simulation, une couleur et/ou une forme et/ou une position d'une partie visible ou invisible de la bouche, de préférence d'une mâchoire, d'une couronne dentaire et/ou d'une racine dentaire et/ou de la gencive du patient, et on détermine la scène dentaire virtuelle de manière qu'elle reproduise ladite couleur et/ou forme et/ou position.

Un tel procédé selon l'invention peut être en particulier utilisé pour simuler :
- l'effet d'un ou plusieurs appareils orthodontiques sur les dents du patient, notamment afin de choisir celui qui lui convient le mieux ;
- l'effet d'une application d'une consigne.

Le procédé peut être utilisé, notamment à des fins pédagogiques, pour visualiser l'effet d'une modification de la fréquence et/ou de la durée et/ou de la technique de brossage des dents, ou l'effet d'un retard dans un changement de gouttière orthodontique et/ou d'un retard pour prendre rendez-vous chez le professionnel des soins dentaires.

Par exemple, il peut être utilisé pour simuler, à l'instant de simulation, la couleur et/ou la position des dents du patient. Notamment, il peut être utilisé pour visualiser l'effet d'un traitement de blanchiment des dents ou l'effet de la cigarette.

Un procédé selon l'invention peut être encore notamment utilisé pour indiquer un emplacement cible de l'arcade dentaire, en particulier sur une ou plusieurs dents.

### Dispositif

Le procédé peut être mis en œuvre avec un dispositif.

Un tel dispositif comporte
- une caméra configurée pour mettre en œuvre l'étape A) ;
- une unité de traitement configurée pour mettre en œuvre l'étape B) ;
- des moyens de présentation configurés pour mettre en œuvre l'étape C), à savoir un écran tel que décrit précédemment ;
l'unité de traitement, la caméra et les moyens de présentation étant configurés pour communiquer entre eux pour mettre en œuvre lesdites étapes.

De préférence, la caméra est configurée de manière à acquérir une succession d'images actualisées de la scène dentaire réelle et transmettre lesdites images actualisées à l'unité de traitement.

Dans un mode de réalisation, la scène dentaire virtuelle est présentée en transparence sur la représentation de la scène dentaire réelle dans l'image actualisée.

Le dispositif comporte un téléphone portable ou une tablette, dans lequel la caméra et les moyens de présentation, de préférence la caméra, les moyens de présentation et l'unité de traitement sont intégrés.

La représentation de la scène dentaire réelle est une image affichée sur l'écran du téléphone portable ou de la tablette.

Comme on le verra plus en détail dans la suite de la description, l'observateur peut ainsi observer la représentation de la scène dentaire réelle sur l'image actualisée et la scène dentaire virtuelle, de préférence en transparence, sur la représentation de la scène dentaire réelle sur l'image actualisée. Il peut ainsi immédiatement avoir accès aux informations contenues dans la scène dentaire virtuelle, présentées dans l'environnement de la scène dentaire réelle.

### Définitions

Un « patient » est une personne pour laquelle un procédé selon l'invention est mis en œuvre, indépendamment du fait que cette personne suive un traitement dentaire ou non.

Une « situation dentaire » définit un ensemble de caractéristiques relatives à une arcade d'un patient à un instant, par exemple la position des dents, leur forme, la position d'un appareil orthodontique, etc. à cet instant.

Par « professionnel des soins dentaires », on entend toute personne qualifiée pour prodiguer des soins dentaires, ce qui inclut un orthodontiste et un dentiste.

L'opérateur est le patient.

Un « appareil orthodontique » est un appareil porté ou destiné à être porté par un patient. Un appareil orthodontique peut être destiné à un traitement thérapeutique ou prophylactique, mais également à un traitement esthétique. Un appareil orthodontique peut être en particulier un appareil à arc et attaches, ou une gouttière orthodontique. Une telle gouttière s'étend de manière à suivre les dents successives de l'arcade sur laquelle elle est fixée. Elle définit une goulotte de forme générale en « U ». La configuration d'un appareil orthodontique peut être en particulier déterminée pour assurer sa fixation sur les dents, mais également en fonction d'un positionnement cible souhaité pour les dents. Plus précisément, la forme est déterminée de manière que, dans la position de service, l'appareil orthodontique exerce des contraintes tendant à déplacer les dents traitées vers leur positionnement cible (appareil orthodontique actif), ou à maintenir les dents dans ce positionnement cible (appareil orthodontique passif, ou « de contention »).

Par "image", on entend une image en deux dimensions. Une image est formée de pixels. Les « images actualisées » sont des images prises à un instant dit « actualisé ». Elles sont de préférence extraites d'un film pris par une caméra.

Par « arcade », on entend tout ou partie d'une arcade dentaire. Par « image d'une arcade », ou « modèle d'une arcade », on entend ainsi une représentation en 2 ou 3 dimensions, respectivement, de tout ou partie de ladite arcade.

Une «scène» est constituée par un ensemble d'éléments qui peuvent être observés simultanément. Une « scène dentaire » est une scène comportant une arcade.

Une « scène réelle » est constituée d'éléments physiques. Une scène dentaire réelle comporte donc une arcade.

Une «scène virtuelle» est constituée de représentations d'éléments, notamment des représentations
- d'un élément physique de la scène dentaire réelle correspondante (c'est-à-dire de la scène dentaire réelle représentée sur l'image actualisée à partir de laquelle la scène dentaire virtuelle a été déterminée) ou
- d'une autre information, ou « indicateur ».

Une représentation d'un élément physique n'est pas nécessairement réaliste.

La présentation d'une scène dentaire virtuelle est « en transparence » sur une image actualisée représentant une scène dentaire réelle lorsqu'elle permet à l'opérateur d'apercevoir l'image actualisée à travers ladite scène dentaire virtuelle.

Une scène dentaire virtuelle est affichée « en superposition » avec une représentation d'une scène dentaire réelle sur une image actualisée lorsqu'elle comporte des représentations d'éléments physiques de la scène dentaire réelle qui sont présentées de manière que les contours desdites représentations, éventuellement transparents, se superposent aux contours desdites représentations desdits éléments physiques sur l'image actualisée. La superposition apparaît ainsi réaliste.

« L'emplacement » d'un élément de la scène dentaire virtuelle fait référence à sa position par rapport aux éléments de la scène dentaire réelle ou de la représentation de ces éléments sur l'image actualisée lorsque la scène dentaire virtuelle est présentée à l'étape C).

Il faut interpréter "comprendre " ou "comporter " ou "présenter " ou « représenter » de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente un exemple de dispositif selon l'invention ;
- la figure 2 représente la superposition d'une scène dentaire virtuelle sur une image actualisée.

Des références identiques sont utilisées pour désigner des organes identiques ou analogues sur les différentes figures.

### Description détaillée

### Dispositif

Un dispositif 10 selon l'invention comporte une caméra 16, une unité de traitement informatique 12 et des moyens de présentation 18. La caméra 16, l'unité de traitement 12 et les moyens de présentation sont pourvus de moyens leur permettant de communiquer entre eux.

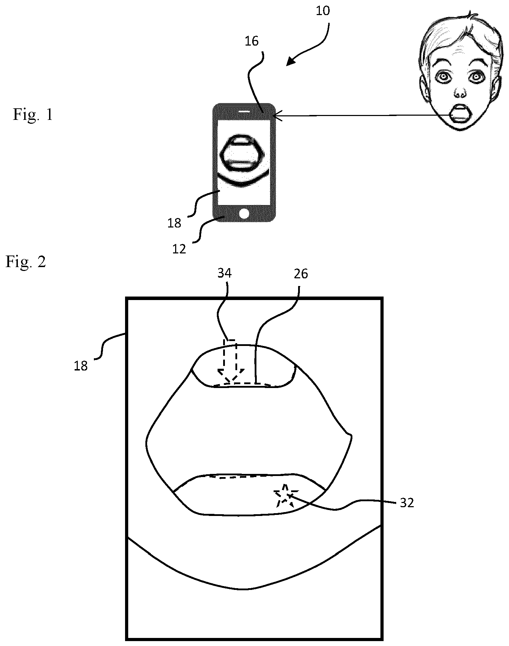

Comme représenté sur la figure 1, la caméra 16 et éventuellement l'unité de traitement 12 et/ou les moyens de présentation sont intégrés dans un téléphone portable ou dans une tablette.

La caméra 16 est destinée à l'acquisition d'images actualisées représentant la scène dentaire réelle observée par l'opérateur sur l'écran d'un téléphone portable (figure 1) ou d'une tablette.

Par « moyens de présentation », on entend tout moyen configuré pour recevoir, de l'unité de traitement 12, une scène dentaire virtuelle et faire apparaître ladite scène dentaire virtuelle sur l'image actualisée.

L'image actualisée et la scène dentaire virtuelle sont présentées sur l'écran, en superposition, afin que l'opérateur voie, en observant cet écran, à la fois l'image actualisée représentant la scène dentaire réelle, mais aussi la scène dentaire virtuelle.

La caméra est immobilisée par rapport à l'écran, par exemple quand la caméra et l'écran sont intégrés dans un téléphone portable (figure 1) ou une tablette.

L'unité de traitement 12 peut comporter les moyens électroniques conventionnels de tout ordinateur, et en particulier une unité centrale, un programme et une mémoire.

Dans un mode de réalisation, le dispositif comporte encore une interface permettant à un opérateur de paramétrer la scène dentaire virtuelle. De préférence, l'interface est configurée pour qu'un opérateur puisse modifier un instant de simulation déterminant la scène dentaire virtuelle. Le contenu de la scène dentaire virtuelle peut en particulier être constitué par la représentation d'éléments de la scène dentaire réelle sous une apparence et/ou à une position simulées pour l'instant de simulation futur.

Par exemple, dans un mode de réalisation, l'écran est un écran tactile et l'opérateur peut modifier l'instant de simulation en entrant une date dans un champ de saisie ou en déplaçant un ou plusieurs doigts sur l'écran. Par exemple un déplacement vers la droite de l'écran peut conduire à avancer vers le futur et un déplacement vers la gauche de l'écran peut conduire à reculer dans le passé. La scène dentaire virtuelle présentée s'adapte en conséquence, de préférence en temps réel.

L'interface peut être également un organe mécanique, par exemple un bouton ou une mollette.

L'unité de traitement détermine les conditions dans lesquelles la scène dentaire virtuelle doit être présentée pour apparaître, à l'opérateur, en superposition avec la scène dentaire réelle.

La scène dentaire virtuelle est présentée sur l'image actualisée affichée sur un écran ; l'opérateur observe l'écran et voit donc ce qui est observé par la caméra. De simples opérations, comme une mise à l'échelle, une correction des effets de perspective ou un recalage sur l'image actualisée, peuvent suffire pour assurer une superposition réaliste de la scène dentaire virtuelle sur l'image actualisée, par exemple en utilisant des marques caractéristiques, comme décrit ci-après.

### Procédé

Sur la figure 2, les éléments de la scène dentaire virtuelle sont représentés en trait interrompu.

Dans un mode de réalisation préféré, une scène dentaire virtuelle comporte une représentation, de préférence réaliste, d'un ou plusieurs éléments de la scène dentaire réelle correspondante. En particulier, de préférence, elle comporte une représentation de l'arcade.

Comme représenté sur la figure 2, la scène dentaire virtuelle peut par exemple représenter les contours 26 des dents et sa projection sur les dents peut faciliter la détection de déplacements ou de déformations des dents.

La scène dentaire virtuelle peut comporter la représentation d'un ou plusieurs éléments physiques qui ne sont pas dans la scène dentaire réelle, par exemple un appareil orthodontique ou un article décoratif 32, par exemple positionné sur la scène dentaire virtuelle de manière à apparaître, sur la scène dentaire réelle, à un emplacement (« emplacement cible ») où l'appareil orthodontique ou l'article décoratif, respectivement, devrait être disposé par le professionnel des soins dentaires ou par le patient.

La représentation d'un élément physique peut être réaliste, par exemple être constituée des contours de l'élément physique considéré, ou symbolique.

La représentation des éléments physiques de la scène dentaire virtuelle est bidimensionnelle.

La scène dentaire virtuelle peut également comporter, en complément ou alternativement à la représentation d'éléments physiques un ou plusieurs indicateurs 34.

Un indicateur est un élément d'une scène dentaire virtuelle qui ne représente pas, même symboliquement, un élément physique de la scène dentaire réelle.

Un indicateur peut être en particulier un texte ou un symbole (un point, un trait, une flèche, un aplat de couleur...).

Dans un mode de réalisation, l'indicateur pointe sur un emplacement sur lequel l'opérateur doit apporter un soin particulier, par exemple un brossage. L'indicateur peut être une image, de préférence ludique, par exemple un dessin d'un monstre ou d'un microbe, sur lequel l'opérateur doit appliquer un outil, par exemple une brosse à dent. Un tel indicateur améliore l'apprentissage du brossage des dents, par exemple.

Sur la figure 2, l'indicateur 34 pointe vers une région dentaire qui a été abrasée.

Les indicateurs sont par exemple stockés dans une base de données dans la mémoire 24 de l'unité de traitement.

La nature et/ou l'emplacement et/ou l'apparence d'un indicateur peut être déterminée en fonction du contexte, et en particulier en fonction de la distance à un autre élément de la scène dentaire virtuelle et/ou en fonction d'un objectif poursuivi. Par exemple, il apparaîtra avec une apparence fonction de la distance à une dent particulière ou fonction du risque pour le patient.

Le contexte est défini par les conditions dans lesquelles le procédé est utilisé.

Par exemple, on identifie un élément de la scène dentaire virtuelle qui ne respecte pas un critère d'évaluation, par exemple parce qu'il dépasse un seuil de risque, puis on attire l'attention vers cet élément dans la scène dentaire virtuelle. Par exemple, la couleur de l'élément peut être modifiée si l'analyse du contexte montre que la situation devient dangereuse.

La scène dentaire virtuelle est déterminée en fonction d'un instant de simulation futur. En particulier, elle peut comporter une représentation, de préférence réaliste, d'un ou plusieurs éléments de la scène dentaire réelle correspondante dans une configuration future.

La scène dentaire virtuelle comporte une représentation de l'arcade et/ou une représentation d'un appareil orthodontique porté par les dents de l'arcade telle qu'anticipée pour un instant de simulation futur.

La simulation à un instant de simulation futur peut être réalisée par tout logiciel de simulation ou par exemple par un professionnel des soins dentaires.

La scène dentaire virtuelle peut par exemple représenter les contours des dents, le positionnement d'un appareil orthodontique ou la couleur des dents à l'instant de simulation. La présentation de la scène dentaire virtuelle sur la scène dentaire réelle, de préférence en transparence sur la scène dentaire réelle, permet ainsi de facilement détecter l'évolution entre l'instant actualisé et l'instant de simulation.

La scène dentaire virtuelle est affichée sur un écran.

L'écran est l'écran d'un téléphone portable ou d'une tablette ; l'opérateur peut ainsi simultanément observer sur l'écran l'image actualisée et la scène dentaire virtuelle.

La scène dentaire virtuelle peut être suffisamment transparente pour laisser apparaître l'image actualisée sous elle.

Alternativement, la scène dentaire virtuelle peut être opaque, de manière à ne pas laisser apparaître l'image actualisée qui s'étend derrière ou sous elle. L'opacité de la scène dentaire virtuelle est particulièrement avantageuse pour simuler une situation dentaire future, par exemple pour visualiser le blanchiment des dents sous l'effet d'un traitement à cet effet, ou le déplacement d'une dent, par exemple sous l'effet d'un traitement orthodontique ou en l'absence d'un traitement orthodontique, par exemple pour visualiser un déchaussement d'une dent. Lorsque la scène dentaire virtuelle est présentée sur l'image actualisée, elle peut être ajoutée au-dessus de l'image actualisée ou, de manière équivalente, l'image actualisée peut être remplacée par une image retraitée informatiquement pour faire apparaître la scène dentaire virtuelle.

Par « présentation de la scène dentaire virtuelle en superposition sur la représentation de la scène dentaire réelle dans l'image actualisée », il faut donc inclure toute présentation faisant apparaître la scène dentaire virtuelle sur la représentation de la scène dentaire réelle, y compris l'affichage d'une image retraitée.

La scène dentaire virtuelle est présentée en superposition avec la représentation de la scène dentaire réelle sur l'image actualisée, ce qui facilite la comparaison. Autrement dit, les éléments de la scène dentaire virtuelle qui correspondent à des éléments de la scène dentaire réelle sont sensiblement exactement superposés à ces derniers ou aux représentations de ces derniers. Notamment lorsque la scène dentaire virtuelle est présentée en transparence, les différences entre la scène dentaire réelle ou sa représentation d'une part et la scène dentaire virtuelle d'autre part apparaissent ainsi clairement à l'opérateur. Les indicateurs lui présentent par ailleurs des informations facilitant l'analyse de la situation.

De préférence, pour présenter la scène dentaire virtuelle en superposition sur la représentation de la scène dentaire réelle sur l'image actualisée, on identifie sur l'image actualisée des marques caractéristiques dont la position par rapport à la scène dentaire virtuelle est connue. Par exemple, on identifie des extrémités de dent ou des formes caractéristiques de dents représentées à la fois sur l'image actualisée et sur la scène dentaire virtuelle.

A l'étape C), on superpose ensuite les représentations de ces marques caractéristiques sur l'image actualisée et dans la scène dentaire virtuelle, ce qui permet de positionner précisément la scène dentaire virtuelle par rapport aux marques caractéristiques représentées sur l'image actualisée.

### Exemple d'application

### Amélioration de l'observance et utilisation à des fins pédagogiques

Dans un mode de réalisation, le procédé est utilisé pour obtenir une meilleure acceptation du traitement par le patient. En particulier, il peut être utilisé pour simuler, de manière réaliste, un traitement dentaire. L'appareil d'acquisition d'images est un téléphone portable ou une tablette.

De préférence, le patient peut modifier l'instant de simulation par interaction avec l'écran, par exemple en déplaçant un curseur virtuel. La scène dentaire virtuelle est adaptée en conséquence, notamment pour tenir compte de l'effet du traitement. En agissant sur le curseur, le patient peut donc simuler le déroulement du traitement, c'est-à-dire visualiser par exemple comment les dents vont se déplacer et/ou changer de couleur, notamment en cas de traitement de blanchiment des dents ou si les dents sont soumises à un agent colorant, par exemple si le patient fume ou boit de grandes quantités de thé ou de café. Le déplacement du curseur lui permet ainsi de simuler une accélération du temps.

Cette simulation facilite la prise de décision par le patient et constitue avantageusement une incitation à suivre le traitement.

Dans un mode de réalisation, le patient peut modifier la valeur d'autres paramètres de la simulation. Par exemple, il peut modifier des valeurs de paramètres du traitement.

Par exemple, il peut simuler
- l'effet d'un ou plusieurs appareils orthodontiques, notamment afin de choisir celui qui lui convient le mieux ;
- l'effet du respect plus ou moins strict des consignes reçues, notamment pour visualiser l'effet d'une modification de la fréquence et/ou de la durée et/ou de la technique de brossage des dents, ou l'effet d'un retard dans un changement de gouttière orthodontique et/ou d'un retard pour prendre rendez-vous chez le professionnel des soins dentaires.

Il peut ainsi simuler l'effet d'une mauvaise observance de la prescription médicale, ce qui est facteur d'amélioration de l'observance.

Dans un mode de réalisation, la scène dentaire virtuelle fait apparaître des avertissements, de préférence sous la forme d'un message ou d'une mise en évidence d'un élément de la scène dentaire virtuelle, par exemple en faisant apparaitre en rouge une zone qui doit être brossée plus soigneusement.

Bien entendu, l'invention n'est pas limitée aux modes de réalisation décrits ci-dessus et représentés, mais telle que définie dans les revendications.

## Revendications

1. Procédé d'analyse d'une situation dentaire réelle d'un patient, à des fins de simulation, le procédé comportant les étapes successives suivantes :
A) à un instant actualisé, acquisition d'une image actualisée représentant une scène dentaire réelle telle qu'observée par un opérateur ;
B) détermination d'une scène dentaire virtuelle en fonction de la représentation de la scène dentaire réelle sur l'image actualisée, la scène dentaire virtuelle comportant une représentation de l'arcade du patient telle qu'anticipée pour un instant de simulation futur et/ou une représentation d'un appareil orthodontique porté par les dents de l'arcade telle qu'anticipée pour un instant de simulation futur, l'instant de simulation étant postérieur à l'instant actualisé de plus de 1 jour ;
C) affichage de l'image actualisée sur un écran et présentation de la scène dentaire virtuelle en superposition avec la représentation de la scène dentaire réelle sur l'image actualisée affichée sur l'écran, en transparence ou non sur ladite représentation,
l'acquisition étant effectuée au moyen d'un téléphone portable (10) ou d'une tablette, procédé dans lequel le patient acquiert ladite image actualisée à l'étape A), et observe l'écran (18) à l'étape C).

2. Procédé selon la revendication 1, dans lequel l'instant de simulation est déterminé avant l'étape A) ou avant l'étape B) par interaction du patient avec l'écran.

3. Utilisation d'un procédé selon l'une quelconque des revendications précédentes, pour simuler :
- l'effet d'un ou plusieurs appareils orthodontiques ;
- l'effet d'un traitement futur, thérapeutique ou non, de préférence pour modifier la couleur ou la position des dents ;
- l'effet d'une modification de la fréquence et/ou de la durée et/ou de la technique de brossage des dents, ou l'effet d'un retard dans un changement de gouttière orthodontique,
- l'effet d'un traitement de blanchiment des dents ou l'effet de la cigarette.

## Patentansprüche

1. Verfahren zur Analyse einer tatsächlichen Zahnsituation eines Patienten zu Simulationszwecken, wobei das Verfahren die folgenden aufeinanderfolgenden Schritte aufweist:
A) Erfassen eines aktualisierten Bilds, das eine tatsächliche Dentalszene darstellt, wie von einem Operateur betrachtet, zu einem aktualisierten Zeitpunkt;
B) Bestimmen einer virtuellen Dentalszene in Abhängigkeit von der Darstellung der tatsächlichen Dentalszene auf dem aktualisierten Bild, wobei die virtuelle Dentalszene eine Darstellung des Zahnbogens des Patienten, wie für einen zukünftigen Simulationszeitpunkt vorausgesehen, und/oder eine Darstellung einer orthodontischen Vorrichtung, die von den Zähnen des Zahnbogens getragen wird, wie für einen zukünftigen Simulationszeitpunkt vorausgesehen, aufweist, wobei der Simulationszeitpunkt um mehr als 1 Tag hinter dem aktualisierten Zeitpunkt liegt;
C) Anzeigen des aktualisierten Bilds auf einem Bildschirm und Darstellen der virtuellen Dentalszene in Überlagerung mit der Darstellung der tatsächlichen Dentalszene auf dem aktualisierten Bild, das auf dem Bildschirm angezeigt wird, in Transparenz auf der Darstellung oder nicht,
wobei das Erfassen mittels eines Mobiltelefons (10) oder eines Tablets erfolgt, wobei bei dem Verfahren der Patient das aktualisierte Bild bei Schritt A) erfasst und den Bildschirm (18) bei Schritt C) betrachtet.

2. Verfahren nach Anspruch 1, wobei der Simulationszeitpunkt vor dem Schritt A) oder vor dem Schritt B) durch Interaktion des Patienten mit dem Bildschirm bestimmt wird.

3. Nutzung eines Verfahrens nach einem der vorhergehenden Ansprüche zur Simulation:
- der Wirkung einer oder mehrerer orthodontischer Vorrichtungen;
- der Wirkung einer zukünftigen therapeutischen oder nicht therapeutischen Behandlung, vorzugsweise zur Änderung der Farbe oder Position der Zähne;
- der Wirkung einer Änderung der Häufigkeit und/oder Dauer und/oder Technik des Zähneputzens oder der Wirkung einer Verspätung bei einem Wechseln einer orthodontischen Schiene,
- der Wirkung einer Zahnaufhellungsbehandlung oder der Wirkung der Zigarette.

## Claims

1. Method for analysing a real dental situation of a patient, for simulation purposes, the method comprising the following successive steps:
A) at a current time, acquiring a current image representing a real dental scene as observed by an operator;
B) determining a virtual dental scene based on the representation of the real dental scene on the current image, the virtual dental scene comprising a representation of the patient's arch as anticipated for a future simulation time and/or a representation of an orthodontic device worn by the teeth of the arch as anticipated for a future simulation time, the simulation time being later than the current time by more than 1 day;
C) displaying the current image on a screen and presenting the virtual dental scene superimposed with the representation of the real dental scene on the current image displayed on the screen, transparently or not transparently over said representation,
the acquisition being performed by way of a mobile telephone (10) or a tablet, in which method the patient acquires said current image in step A), and looks at the screen (18) in step C) .

2. Method according to Claim 1, wherein the simulation time is determined before step A) or before step B) by the patient interacting with the screen.

3. Use of the method according to either one of the preceding claims to simulate:
- the effect of one or more orthodontic devices;
- the effect of a therapeutic or nontherapeutic future treatment, preferably to modify the colour or the position of the teeth;
- the effect of modifying the frequency and/or duration and/or technique of brushing the teeth, or the effect of a delay in changing an orthodontic aligner,
- the effect of a tooth-whitening treatment or the effect of cigarettes.
